# EUROPÄISCHE PATENTSCHRIFT

(11) **EP 1 051 105 B1**
(45) Veröffentlichungstag und Bekanntmachung des Hinweises auf die Patenterteilung: **28.04.2004**
(21) Anmeldenummer: 99907449.5
(22) Anmeldetag: 29.01.1999
(51) Int. Cl.: A61B 5/0225

(54) **VERFAHREN ZUR BESTIMMUNG DES BLUTDRUCKS UND BLUTDRUCKMESSGERÄT**
METHOD AND DEVICE FOR MEASURING BLOOD PRESSURE
PROCEDE ET DISPOSITIF POUR MESURER LA TENSION ARTERIELLE

(30) Priorität: 30.01.1998 DE 19803530
(43) Veröffentlichungstag der Anmeldung: 15.11.2000
(73) Patentinhaber: Braun GmbH, 61476 Kronberg (DE)
(72) Erfinder: FREUND, Dirk, D-65779 Kelkheim (DE); GIERSIEPEN, Martin, D-61440 Oberursel (DE); HARTTMANN, Brigitte, D-65527 Niedernhausen (DE); HOLLINGER, Stefan, D-61476 Kronberg (DE); KRESSMANN, Frank, D-65824 Schwalbach (DE); SCHNAK, Fred, D-61476 Kronberg (DE)
(86) Internationale Anmeldenummer: PCT/EP1999/000573
(87) Internationale Veröffentlichungsnummer: WO 1999/038434

(56) Entgegenhaltungen:
- EP-A- 0 335 179
- EP-A- 0 698 370
- EP-A- 0 721 764
- US-A- 5 577 508

## Beschreibung

Die vorliegende Erfindung betrifft ein Verfahren zur Bestimmung des Blutdrucks, wobei mittels einer Manschette ein Körperglied abgeschnürt wird, ein Manschettendruck verändert und mittels einer Druckerfassungseinrichtung erfaßt wird, Amplituden von erfaßten Pulsschlagoszillationen (nachfolgend "Oszillationen" genannt) des Manschettendrucks bestimmt werden, aus den Amplituden erfaßter Oszillationen das Auftreten einer wahren maximalen Oszillation bestimmt wird und Blutdruckwerte in Abhängigkeit von der wahren maximalen Oszillation bestimmt werden, wobei zwei lineare Regressionen bestimmt werden, von denen eine durch die Amplituden von Oszillationen, die vor der erfaßten maximalen Oszillation erfaßt wurden und die andere durch die Amplituden von Oszillationen, die nach der erfaßten maximalen Oszillation erfaßt wurden, bestimmt wird.

Die Erfindung betrifft ferner ein Blutdruckmeßgerät mit einer Manschette zum Abschnüren eines Körperglieds, einer Drucksteuereinrichtung zur Steuerung eines Manschettendrucks, einer Druckerfassungseinrichtung zur Erfassung des Manschettendrucks, einer Amplituden-Bestimmungseinrichtung zur Bestimmung der Amplituden von Oszillationen des Manschettendrucks, einer Maximum-Berechnungseinrichtung zur Berechnung des Auftretens einer wahren maximalen Oszillation aus den Amplituden erfaßter Oszillationen und einer Blutdruckwerte-Berechnungseinrichtung zur Berechnung von Blutdruckwerten in Abhängigkeit der wahren maximalen Oszillation, wobei zwei lineare Regressionen bestimmbar sind, von denen eine durch die Amplituden von Oszillationen, die vor der erfaßten maximalen Oszillation erfaßt wurden und die andere durch die Amplituden von Oszillationen, die nach der erfaßten maximalen Oszillation erfaßt wurden, bestimmt wird.

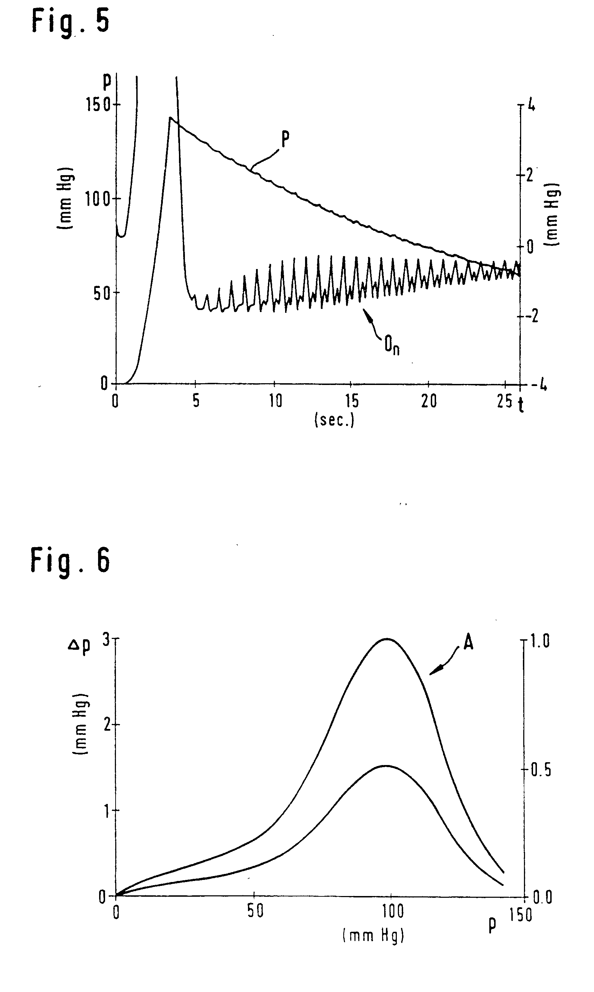

Bei der oszillometrischen Blutdruckmessung werden die Blutdruckwerte aus den Druckschwankungen bestimmt, die bei einer kontinuierlichen Veränderung des Manschettendrucks auftreten und auf den Pulsschlag zurückgehen. Üblicherweise wird der Manschettendruck durch Aufpumpen der Manschette zunächst auf einen ausreichend hohen Wert erhoht und dann für den eigentlichen Meßvorgang kontinuierlich mit einer konstanten Ablaßrate abgelassen. Die während des Ablaßvorganges auftretenden Oszillationen des Manschettendrucks nehmen zunächst zu, erreichen ein Maximum und nehmen danach monoton mit abfallendem Manschettendruck ab. Eine maximale Oszillation. deren Amplitude relativ zu den vorausgegangenen und nachfolgenden Amplituden maximal ist, tritt dann auf, wenn der Manschettendruck einen dem mittleren arteriellen Blutdruck (MAP) entsprechenden Wert erreicht. Bei dem systolischen und dem diastolischen Blutdruckwert tritt jeweils eine Oszillation mit einer Amplitude auf, die einen bestimmten Bruchteil der maximalen Amplitude beträgt. Ausgehend von dem Auftreten der maximalen Oszillation lassen sich dementsprechend abhängig vom Meßort wie beispielsweise Oberarm oder Handgelenk und vom Meßvolumen wie beispielsweise der Manschettengröße, der Schlauchlänge, der Lage der Manschettenblase auf der Ader etc. die Blutdruckwerte bestimmen. Bestimmend für die Meßgenauigkeit ist daher stets die genaue Bestimmung des Auftretens der maximalen Oszillation (vgl. "Theory of the Oscillometric Maximum and the Systolic and Diastolic Detection Ratios" von G. Drzewiecki et al in Annals of Biomedical Engineering, Vol. 22, Seiten 88-96, 1994). Das Auftreten der Oszillationen während eines Ablaßvorganges ist modellhaft in den Figuren 5 und 6 dargestellt, wobei im unteren Teil von Figur 5 neben dem Verlauf des Manschettendrucks die herausgefilterten Oszillationsanteile des Manschettendrucks vergrößert dargestellt sind und in Figur 6 die Hüllkurve über die Oszillationen dargestellt ist.

Ein Problem bei der Blutdruckmessung mittels einer ein Körperglied abschnürenden Manschette ist die Dauer der Messung und die Höhe des Manschettendrucks, die für den Patienten eine Belastung darstellen und deshalb möglichst gering gehalten werden sollten. Zudem wohnt dieser Belastung eine Fehlerquelle inne, da durch Befreiungsbewegungen des Patienten Störimpulse auf den Manschettendruck aufgeprägt werden.

Andererseits besteht bei einer raschen Änderung des Manschettendrucks das Problem, daß die maximale Oszillation nur ungenau bzw. mehr oder weniger zufällig erfaßt werden kann. Der dem mittleren arteriellen Blutdruck entsprechende Manschettendruckwert wird zu einem Zeitpunkt überfahren, bei dem nicht unbedingt ein Pulsschlag und dementsprechend eine Oszillation auftreten muß, so daß bei der Messung nur die jeweils bei einem höheren bzw. niedrigeren Manschettendruck vor und nach der wahren maximalen Oszillation auftretende Druckschwankung erfaßt werden kann. Je größer die Druckänderungsrate ist, desto größer ist der mögliche Meßfehler.

Um auch bei einer raschen Messung ein genaues Ergebnis zu erzielen, wurde bereits vorgeschlagen, aus den Amplituden der tatsächlich erfaßten Oszillationen das Auftreten der wahren maximalen Oszillation zu berechnen, das heißt zu bestimmen, wann bzw. bei welchem Manschettendruck die wahre maximale Oszillation aufgetreten wäre. Hierzu wurde eine bestmöglich an die erfaßten Oszillationen bzw. deren Amplituden schmiegende Hüllkurve berechnet und aus dieser in einem weiteren Schritt die wahre maximale Oszillation bestimmt (vgl. EP 0 787 463 A2). Die Berechnung dieser Hüllkurve ist jedoch verhältnismäßig komplex, bedingt einen großen Rechenaufwand und erfordert einen großen Speicher.

In der US 5577508 werden zwei Geradenannäherungen an die Hüllkurve der Amplitudenmaxima vorgenommen, die aus einer Vielzahl von vorangegangenen Geradenannäherungen durch steten Neigungvergleich hervorgegangen sind. Dabei ist die erfaßte maximale Oszillation Bestandteil der Geradenannäherung. In einem zweiten Schritt werden Amplitudenwerte, die eine bestimmte Schwelle nicht überschreiten nicht mehr berücksichtigt, so daß ein neuer Schnittpunkt aus zwei Annäherungsgeraden entsteht, der seinerseits durch Geradenneigungsvergleiche verbessert werden soll. Dieses Verfahren ist sehr anfällig für Bewegungsartefakte, da hohe Amplitudenausschläge bei gleichzeitiger Bewegung den Schnittpunkt der Geraden stark versetzen können. Zudem ist das Verfahren ungenau, wenn die wahre maximale Amplitude weit von der erfaßten abweicht, da letztlich die Berechnung des Blutdrucks auf einem der Geradenschnittpunkte basiert.

Der vorliegenden Erfindung liegt daher die Aufgabe zugrunde, ein Verfahren zur Bestimmung des Blutdrucks und ein Blutdruckmeßgerät anzugeben, die die Nachteile bekannter Verfahren und Geräte vermeiden. Insbesondere soll mit einem verhältnismäßig kurzen Meßvorgang in einfacher Weise also mit hoher numerischer Effizienz ein ausreichend genaues Meßergebnis bei guter Stabilität gegen Artefakte erzielt werden.

Bei einem Verfahren der eingangs genannten Art wird diese Aufgabe erfindungsgemäß dadurch gelöst, daß das Auftreten der wahren maximalen Oszillation in Abhängigkeit von zwei linearen Regressionen bestimmt wird, von denen eine durch die Amplituden von Oszillationen, die vor der erfaßten maximalen Oszillation erfaßt wurden, und die andere durch die Amplituden von Oszillationen, die nach der erfaßten maximalen Oszillation erfaßt wurden, bestimmt wird. Es wird also mittels einer Regressionseinrichtung eine erste Regressionsgerade durch die Amplitudenwerte von in der Amplitude ansteigenden Oszillationen und eine zweite Regressionsgerade durch die Amplitudenwerte von in der Amplitude kleiner werdenden Oszillationen bestimmt. Der Schnittpunkt der beiden Regressionsgeraden bestimmt dabei lediglich in erster Näherung, zu welchem Zeitpunkt des Meßvorganges bzw. bei welchem Manschettendruck die wahre maximale Oszillation aufgetreten wäre.

Das Auftreten der wahren maximalen Oszillation wird in Abhängigkeit des Schnittpunkts der beiden Regressionsgeraden und dem Auftreten der erfaßten maximalen Oszillation bestimmt Insbesondere kann das Auftreten der wahren maximalen Oszillation als Mittelwert oder als anderer Verhaltniswert von dem mit Hilfe der linearen Regressionen berechneten Auftreten und dem Auftreten der tatsächlich erfaßten maximalen Oszillation, die von den erfaßten die maximale Amplitude aufweist, berechnet werden. Das Auftreten der wahren maximalen Oszillation wird also nicht ausschließlich durch die linearen Regressionen bestimmt, sondern wird darüber hinaus von der tatsächlich erfaßten maximalen Oszillation abhängig gemacht. Dies besitzt den Vorteil, daß während des Meßvorganges aufgetretene mögliche Blutdruckverfälschungen keinen übermäßigen Einfluß auf die Bestimmung der wahren maximalen Oszillation gewinnen und das Meßergebnis verfälschen können. Bewegungen während der Messung können zur Folge haben, daß die Amplituden der Druckschwankungen verändert werden und sich dementsprechend die linearen Regressionen ändern und der Schnittpunkt der beiden Regressionsgeraden weit verschoben werden würde. Ein solcher großer Meßfehler wird vermieden, indem das Auftreten der wahren maximalen Oszillation von zwei Faktoren abhängig gemacht wird, nämlich den beiden linearen Regressionen und der tatsächlich erfaßten maximalen Oszillation. Die tatsächlich (meßtechnisch) erfaßte maximale Oszillation wird also neben dem gebildeten Schnittpunkt der Regressionsgeraden zur Ermittlung der wahren maximalen Oszillation verwendet.

Das Auftreten der maximalen Oszillation kann auch durch die Festlegung eines Verhältniswertes zwischen dem mit Hilfe der linearen Regressionen berechneten und dem tatsächlich erfaßten Auftreten der maximalen Oszillation bestimmt werden, der sowohl invariant als auch kurvenadaptiv gewählt sein kann. Die zuerst beschriebene Verfahrensausbildung, nach der ein Mittelwert gebildet wird, bewirkt jedoch bei einer einfachen Ausführbarkeit eine ausreichende Sicherheit gegen Meßfehler infolge Bewegungsartefakten.

Um aus dem Auftreten der jeweiligen Oszillation, die für den jeweiligen Blutdruckwert, das heißt den mittleren arteriellen, den diastolischen bzw. den systolischen Blutdruck, kennzeichnend ist, den entsprechenden Blutdruckwert zu bestimmen, wird der entsprechende Manschettendruck, der mit der Druckerfassungseinrichtung bei der jeweiligen Oszillation erfaßt wurde, festgestellt. Vorzugsweise wird hierzu ein wahrer Manschettendruck, der frei von Oszillationen ist, berechnet und die Blutdruckwerte aus dem wahren Manschettendruck bestimmt. Der erfaßte Manschettendruck wird also von den überlagerten Oszillationen infolge des Pulses bereinigt. Dies bewirkt eine Bestimmung des Blutdrucks mit erhöhter Genauigkeit. Unabhängig von der Bestimmung des Auftretens der wahren maximalen Oszillation in Abhängigkeit der beiden linearen Regressionen besitzt die Bestimmung des wahren Manschettendrucks den Vorteil, daß der Einfluß von überlagerten Druckschwankungen auf die Bestimmung der Blutdruckwerte beseitigt und eine höhere Genauigkeit bei der Blutdruckmessung erreicht wird.

Vorzugsweise werden im Bereich der Oszillationen wahre Manschettendruckwerte durch Interpolation bestimmt. Vor und / oder nach einer Oszillation erfaßte Manschettendruckwerte werden in den Bereich der jeweiligen Oszillation interpoliert. Gegebenenfalls können die wahren Manschettendruckwerte auch durch Extrapolation von vor und / oder nach einer Oszillation erfaßter Manschettendruckwerte bestimmt werden. Die Heranziehung von Manschettendruckwerten auf beiden Seiten der jeweiligen Oszillation bewirkt jedoch eine genauere und sichere Bestimmung der wahren Manschettendruckwerte.

In Weiterbildung der Erfindung werden in einem weiteren Verfahrensschritt die während des Meßvorgangs erfaßten Daten reduziert. Vorzugsweise wird für jede Oszillation ein Oszillationsmaximum (das einen Maximalwert in jeder einzelnen Oszillation an sich angibt) und ein zugehöriger Fußpunkt der Oszillation bestimmt. Der Fußpunkt ist dabei insbesondere der Druckwert, der der Differenz von dem jeweiligen Oszillationsmaximum und der Oszillationsamplitude entspricht. Der Fußpunkt kann als wahrer Manschettendruckwert zum Zeitpunkt des Oszillationsmaximums verwendet werden. Vorzugsweise wird das jeweilige Oszillationsmaximum, der zugehörige Fußpunkt und der entsprechende Zeitpunkt, an dem das Oszillationsmaximum erfaßt wurde, als Datendreisatz in einer Speichereinrichtung abgespeichert. Die während der jeweiligen Oszillation erfaßte Vielzahl von Meßwerten kann gelöscht und durch den genannten Datendreisatz, ein sogenanntes Triplet ersetzt werden. Die Datenreduktion kann parallel zum Meßvorgang bewirkt werden. Dies besitzt den Vorteil, daß ein Speicher kleinerer Kapazität verwendet werden kann.

Der Fußpunkt einer Oszillation wird vorzugsweise durch lineare Interpolation von vor und nach der Oszillation erfaßter Manschettendruckwerte bewirkt. Insbesondere wird zwischen einem Mittelwert von Manschettendruckwerten, die in einem Zeitfenster vor der Oszillation erfaßt wurden, und dem Mittelwert von Manschettendruckwerten, die in einem Zeitfenster vor einer nachfolgenden Oszillation erfaßt wurden, interpoliert bzw extrapoliert. Das jeweilige Zeitfenster vor einer Oszillation kann in Abhängigkeit von der Pulsfrequenz bestimmt werden, die hierfür mittels einer Pulserfassungseinrichtung erfaßt wird.

Um neben dem mittleren arteriellen Blutdruck auch den systolischen Blutdruck und den diastolischen Blutdruck bestimmen zu können, wird das Auftreten der jeweiligen Oszillation bestimmt, das abhängig vom Meßort und vom Meßvolumen wie beispielsweise der Manschettengröße oder der Schlauchlänge oder dgl. den hohen systolischen Blutdruck und den niedrigen diastolischen Blutdruck festlegt. In Weiterbildung der Erfindung wird das Auftreten dieser jeweiligen Oszillation aus den beiden linearen Regressionen bestimmt, die zur Bestimmung der wahren maximalen Oszillation bereits verwendet wurden. Insbesondere wird die entsprechende Oszillation dadurch bestimmt, daß deren Amplitude in einem bestimmten Verhältnis zur Amplitude der maximalen Oszillation steht. Das Auftreten der den systolischen Blutdruckwert kennzeichnenden Oszillation wird durch die erste Regressionsgerade bestimmt, die durch die Amplitudenwerte der in der Amplitude ansteigenden Oszillationen festgelegt ist und vor der maximalen Oszillation auftritt. Die erfaßte maximale Oszillation selbst wird für die Bildung einer der beiden oder beider Regressionsgeraden nicht verwendet. Die den diastolischen Blutdruckwert kennzeichnende Oszillation wird durch die zweite Regressionsgerade bestimmt, die durch die Amplitudenwerte der in der Amplitude kleiner werdenden Oszillationen festgelegt ist Die beiden linearen Regressionen werden also sowohl für die Bestimmung des mittleren arteriellen Blutdrucks als auch für die Bestimmung des diastolischen und systolischen Blutdrucks verwendet. Die beiden Regressionsgeraden sind ausreichend. Zusätzliche Näherungsverfahren sind unnötig. In vorteilhafter Weise kann hierdurch der Berechnungsaufwand wesentlich reduziert werden.

Zur Bestimmung der Regressionsgeraden werden in Weiterbildung der Erfindung Oszillationen ausgewählt, die in einem begrenzten Bereich um einen jeweiligen systolischen und diastolischen Erwartungswert erfaßt wurden Es werden also vorzugsweise nicht sämtliche Amplitudenwerte auf der jeweiligen Seite der maximalen Oszillation verwendet. Dies reduziert den Aufwand bei der Regressionsbestimmung und verringert den Einfluß wenig aussagekräftiger Schwankungen. Vorzugsweise wird der begrenzte Bereich in Abhangigkeit der Herzfrequenz bestimmt. Vorzugsweise werden für die erste Regression durch die systollschen Oszillationen zumindest 5, vorzugsweise 5 bis 9 Oszillationen verwendet. Für die diastolische Regression werden vorzugsweise zumindest 4, insbesondere 4 bis 6 Oszillationen verwendet. Hierdurch kann die Patientenbelastung durch eine kurze Meßzeit gering gehalten und dennoch aussagekräftige und genaue Ergebnisse erzielt werden.

Bei einem Blutdruckmeßgerät der eingangs genannten Art wird die oben genannte Aufgabe erfindungsgemäß dadurch gelöst, daß die Maximum-Berechnungseinrichtung eine Einrichtung aufweist, die das Auftreten der wahren maximalen Oszillation in Abhängigkeit vom Schnittpunkt zweier linearer Regressionen bestimmt, von denen eine durch die Amplituden von Oszillationen, die vor der erfaßten maximalen Oszillation erfaßt wurden, und die andere durch die Amplituden von Oszillationen, die nach der erfaßten maximalen Oszillation erfaßt wurden, bestimmt wird. Die linearen Regressionen werden mittels einer Regressionseinrichtung zur Bildung von linearen Regressionen bereitgestellt. Zusätzlich ist die Einrichtung derart ausgebildet, daß zur Berechnung des Auftretens der wahren maximalen Oszillation parallel auch die erfaßte maximale Oszillation gleiche Berücksichtigung durch einen Verhältniswert mit dem Schnittpunkt findet. Damit ist die wahre maximale Oszillation sowohl in der Nähe des Schnittpunkts der Regressionsgeraden als auch der erfaßten maximalen Oszillation, ohne mit einem von beidem identisch zu sein. Die Anfälligkeit für Artefakte ist durch diese zwei gleichrangigen Einflußgrößen klein.

Zur Bestimmung des wahren Manschettendrucks ist eine Oszillations-Filtereinrichtung zur Filterung der Oszillationen vorgesehen Die wahren Manschettendruckwerte, die den Manschettendruck frei von Oszillationen wiedergeben, werden der Blutdruckwerte-Berechnungseinrichtung bereitgestellt, die die Blutdruckwerte aus dem wahren Manschettendruck bestimmt.

In Weiterbildung der Erfindung ist eine Datenreduktionseinrichtung zur Reduktion von Daten des erfaßten Manschettendrucks vorgesehen. Die Datenreduktionseinrichtung arbeitet vorzugsweise parallel (bzw. Online) zur Datenerfassung, so daß nur eine verminderte Anzahl von Daten abgespeichert zu werden braucht und eine Speichereinrichtung zur Speicherung der Manschettendruckdaten verhältnismäßig klein ausgebildet sein kann Vorzugsweise weist die Datenreduktionseinrichtung eine Fußpunkt-Bestimmungseinrichtung zur Bestimmung eines jeweils zu einem Oszillationsmaximum zugehörigen Fußpunkts auf. Die Fußpunkt-Bestimmungseinrichtung besitzt vorzugsweise eine Mittelwerte-Bestimmungseinrichtung zur Bestimmung von Mittelwerten von vor und nach einer Oszillation erfaßten Manschettendruckwerten und eine Berechnungseinheit zur Berechnung des jeweiligen Fußpunktes in Abhängigkeit der Mittelwerte. Zweckmäßigerweise ist eine Approximationseinrichtung. vorzugsweise eine Interpolationseinrichtung zur linearen Interpolation von vor und nach einer Oszillation erfaßten Manschettendruckwerten. insbesondere der der Mittelwerte, in den Bereich der Oszillation vorgesehen. Die Interpolationseinrichtung bestimmt dabei eine Gerade zwischen den erfaßten Manschettendruckwerten bzw. deren Mittelwerten und bestimmt den Wert der Geraden zum Zeitpunkt des Auftretens des Oszillationsmaximums. Der so ermittelte Fußwert entspricht zumindest näherungsweise der Differenz zwischen dem Oszillationsmaximum und der Amplitude der Oszillation. Zur Bestimmung des Oszillationsmaximums ist eine entsprechende Einrichtung vorgesehen, die ein lokales Maximum der von der Druckerfassungseinrichtung erfaßten Manschettendruckwerte bezogen auf den Verlauf des Meßvorganges bestimmt.

Die im Wege der Datenreduktion bereitgestellten Oszillationsmaxima und die zugehörigen Fußpunkte werden zusammen mit den jeweiligen Zeitpunkten, zu denen die entsprechenden Oszillationsmaxima aufgetreten sind, in einer entsprechend ausgebildeten Speichereinrichtung als Datendreisatz abgespeichert. Die entsprechenden Zeitpunkte werden von einer Zeiterfassungsvorrichtung erfaßt und der Speichereinrichtung entsprechend bereitgestellt.

Zur Bestimmung der beiden Regressionsgeraden weist die Maximum-Berechnungseinrichtung in Weiterbildung der Erfindung eine Auswahleinrichtung zur Auswahl von Oszillationen auf. Die Auswahleinrichtung besitzt vorzugsweise eine Erwartungswert-Berechnungseinrichtung zur Berechnung eines systolischen und eines diastolischen Erwartungswerts und eine Bereichs-Berechnungseinrichtung zur Berechnung je eines Bereichs um den systolischen und den diastolischen Erwartungswert insbesondere in Abhängigkeit der Herzfrequenz.

Um den systolischen bzw. den diastolischen Blutdruckwert bestimmen zu können, besitzt vorzugsweise die Blutdruckwert-Berechnungseinrichtung eine Einrichtung, die ein Auftreten einer Oszillation, die den entsprechenden Blutdruckwert kennzeichnet, aus den beiden Regressionsgeraden bestimmt. Insbesondere wahlt die Einrichtung einen Punkt bzw. einen Wert aus jeweils einer der Regressionsgeraden der zu dem Wert der wahren maximalen Oszillation in einem vorgegebenen Verhältnis steht und bestimmt den zugehörigen Zeitpunkt. Die Blutdruckwerte-Berechnungseinrichtung bestimmt dann in einem weiteren Schritt, welcher Manschettendruckwert zusammen mit dem entsprechenden Zeitpunkt in der Speichereinrichtung abgelegt ist. Dieser Manschettendruckwert entspricht dann dem jeweiligen Blutdruckwert.

Diese und weitere Merkmale gehen außer aus den Ansprüchen auch aus der Beschreibung und den zugehörigen Zeichnungen hervor. Nachfolgend wird ein Ausführungsbeispiel der Erfindung anhand zugehöriger Zeichnungen näher erläutert. In diesen zeigen:
- Figur 1: den Aufbau eines Blutdruckmeßgerätes gemäß einer bevorzugten Ausführung der Erfindung in einer schematischen Darstellung,

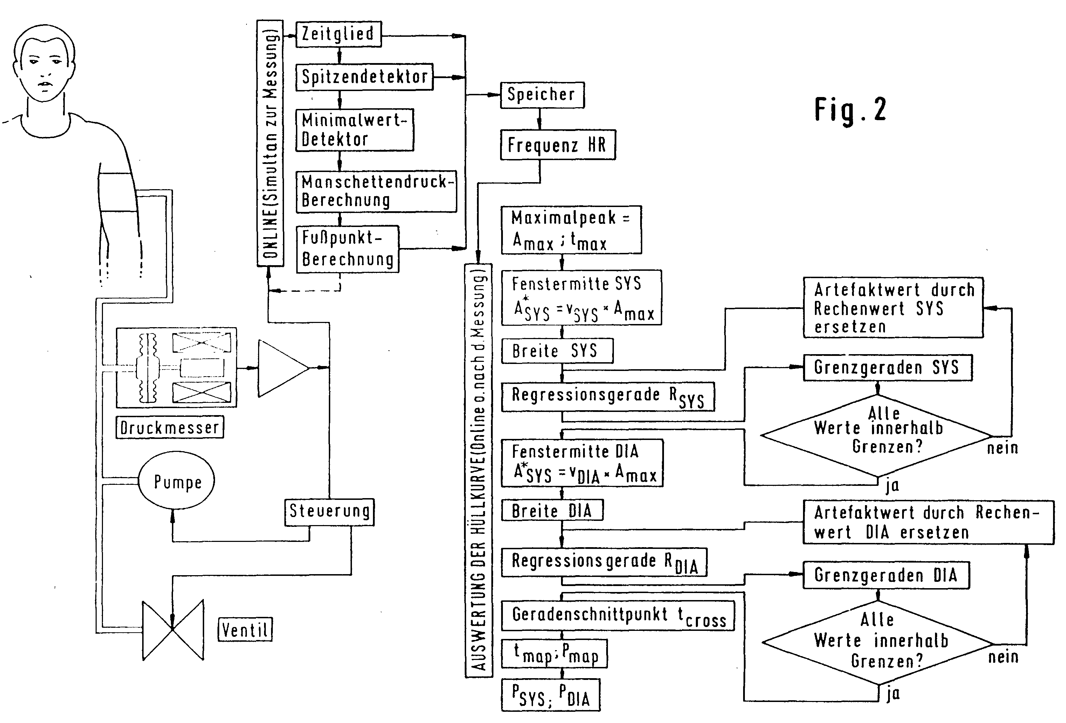

- Figur 2: ein Funktionsschaubild des Blutdruckmeßgerätes gemäß Figur 1,
- Figur 3a: ein Diagramm des Manschettendrucks über der Zeit, das in einem Ausschnitt den Verlauf des Manschettendrucks während eines Meßvorgangs zeigt,
- Figur 3b: einen vergrößerten Ausschnitt des Manschettendruckverlaufes gemäß Figur 3a, der zwei Oszillationen des Manschettendrucks während des Ablaßvorganges zeigt,
- Figur 4: ein Diagramm der Oszillationsamplituden über der Zeit, das den Verlauf der Oszillationsamplituden während eines Meßvorganges in einem Ausschnitt zeigt,
- Figur 5: den Verlauf des Manschettendrucks sowie der hochpaßgefilterten Oszillationsanteile des Manschettendrucks in einer modelthaften Darstellung, wobei beide Verläufe in einem gemeinsamen Diagramm gezeigt sind, und
- Figur 6: ein Diagramm, das die Hüllkurve zu den Oszillationen während des Ablaßvorganges über dem absoluten Manschettendruck zeigt, wobei die Hüllkurve einmal dimensionsbehaftet und in einer zweiten Darstellung dimensionslas normiert dargesteilt ist.

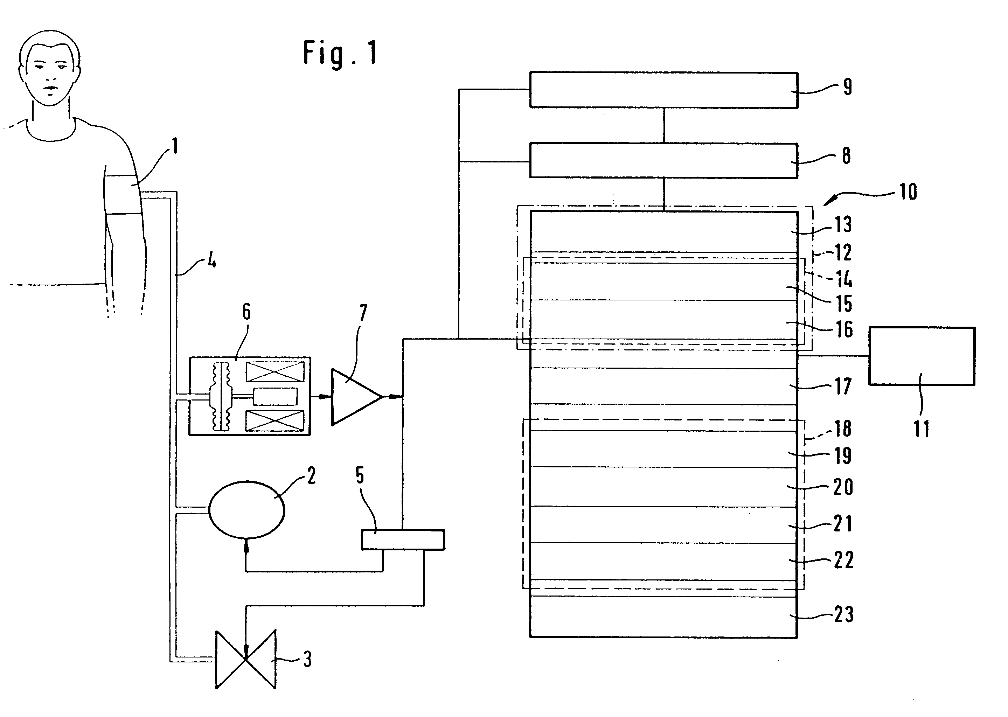

Das Blutdruckmeßgerät ist als Oberarm-Meßgerät ausgebildet und besitzt eine Manschette 1, die um den linken Oberarm eines Patienten angelegt und mit einem Fluid, insbesondere Luft, aufgepumpt werden kann, so daß der Oberarm des Patienten abgeschnürt wird (vgl. Figur 1). Das Blutdruckmeßgerät könnte auch als Handgelenks- oder ggf. Finger-Meßgerät ausgebildet sein. Unter Abschnüren eines Körpergliedes werden hier alle Maßnahmen verstanden, durch die ein im Körperglied verlaufendes Blutgefäß (Arterie) durch äußeren Druck vorübergehend stark verformt wird, so daß eine oszillometrische Messung ermöglicht wird. Zum Aufpumpen der Manschette 1 ist eine Pumpe 2 vorgesehen, die von einem nicht näher dargestellten Antrieb angetrieben wird. Um die Luft aus der Manschette 1 ablassen zu konnen, ist ein Ablaßventil 3 vorgesehen, das wie die Pumpe 2 über eine Leitung 4 mit der Manschette 1 verbunden ist. Der Aufpump- sowie der Ablaßvorgang, der später noch näher erläutert werden wird, wird mittels einer Drucksteuereinrichtung 5 bewirkt, die den Manschettendruck steuert. Die Drucksteuereinrichtung 5 ist hierzu mit der Pumpe 2 und dem Ablaßventil 3 verbunden, um diese anzusteuern und zu betätigen. Die Drucksteuereinrichtung 5 ist ferner mit einer Druckerfassungseinrichtung 6 verbunden, die wiederum mit der Manschette 1 verbunden ist und den Manschettendruck der Manschette 1 erfaßt. Ein den Manschettendruck kennzeichnendes Signal der Druckerfassungseinrichtung 6 wird über einen Analog/Digital-Wandler und Verstärker 7 bereitgestellt. Mit Hilfe des Signals der Drukkerfassungseinrichtung 6 kann die Drucksteuereinrichtung 5 den Aufpump- und insbesondere den Ablaßvorgang rückkoppelnd steuern und die exakte Einhaltung eines erwünschten Druckverlaufes bewirken.

Für den eigentlichen Meßvorgang ist ein Speicher 8 vorgesehen, in dem die von der Druckerfassungseinrichtung 6 jeweils erfaßten Manschettendruckwerte abgespeichert werden können. Die Zeit wird während eines Meßvorganges mit Hilfe einer Zeiterfassungsvorrichtung 9 erfaßt.

Zur Verarbeitung und Auswertung der wahrend eines Meßvorganges erfaßten Meßwerte ist eine Auswerteeinheit 10 vorgesehen, deren funktioneller Aufbau später noch näher erläutert werden wird. Das Ergebnis der Blutdruckmessung wird schließlich mittels einer Anzeigeeinheit 11, die mit der Auswerteeinheit 10 verbunden ist, angezeigt.

Zur Bestimmung des Blutdrucks wird zunachst die Manschette 1 relativ rasch von der Pumpe 2 auf einen Manschettendruck aufgepumpt. der über dem erwarteten Blutdruck liegt. Zur eigentlichen Blutdruckmessung wird dann der Manschettendruck kontinuierlich mit einer vorgegebenen festen Ablaßrate reduziert (vgl Figur 5). Wie der Manschettendruckwerteverlauf P in Figur 5 zeigt, sind dem Manschettendruck wahrend des Ablaßvorganges Druckschwankungen bzw. Oszillationen O überlagert. die aus dem infolge des Herzschlags pulsierenden Blutdruck resultieren. Wie die in Figur 5 vergrößert dargestellten, hochpaßgefilterten Oszillationsanteile O des Manschettendrucks und der Verlauf der Oszillationen in Figur 6 zeigen, steigen die Oszillationen zunächst monoton bis zu einer maximalen Oszillation an, wahrend der Manschettendruck kontinuierlich abgelassen wird. Danach sinken die Amplituden der Oszillationen wieder bis zum volligen Verschwinden ab. Figur 3a zeigt den abnehmenden Verlauf der mit Oszillationen überlagerten Manschettendruckwerte P während des Ablaßvorganges in einem vergrößerten Ausschnitt.

Die von der Druckerfassungseinrichtung 6 erfaßten Manschettendruckwerte P werden zunächst in dem Speicher 8 zusammen mit den jeweils zugehörigen Zeitwerten abgelegt, die von der Zeiterfassungseinrichtung 9 bestimmt werden und den Zeitpunkt angeben, zu dem ein jeweiliger Meßwert aufgetreten ist. Um Ungenauigkeiten bei der Bestimmung der Blutdruckwerte zu vermeiden, wird zunächst ein wahrer Manschettendruck, der frei von Oszillationen ist, bestimmt, das heißt die Oszillationsanteile der Manschettendruckwerte werden gefiltert. Hierzu besitzt die Auswerteeinheit 10 eine Oszillations-Filtereinrichtung 12 (vgl. Figur 1), die mittels eines Spitzendetektors 13 zu jeder Oszillation das Oszillationsmaximum, das heißt im Manschettendruckwerteverlauf ein jeweiliges relatives Maximum, und mittels einer Fußpunkt-Bestimmungseinrichtung 14 einen zu dem jeweiligen Oszillationsmaximum zugehörigen Fußpunkt bestimmt.

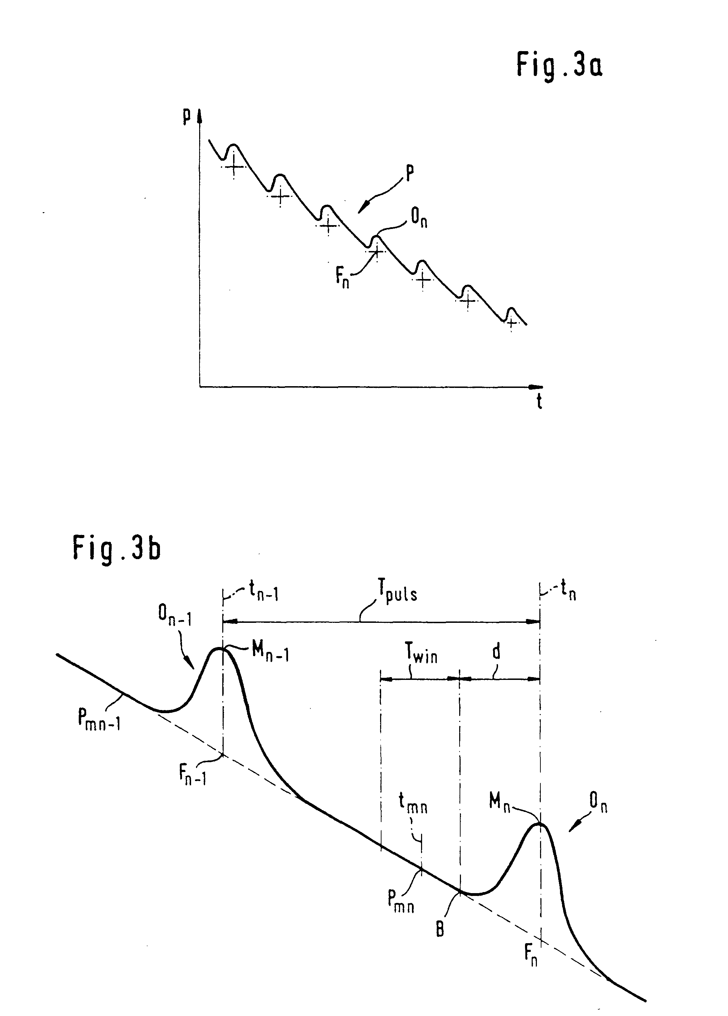

Die Fußpunkt-Bestimmung wird nachfolgend in Verbindung mit Figur 3b näher erläutert, die in einer vergrößerten Darstellung den Verlauf der Manschettendruckwerte P mit zwei aufeinanderfolgenden Oszillationen zeigt.

Zunächst wird vor der Oszillation Oₙ ein Manschettendruckwert bestimmt, der vor der Oszillation Oₙ liegt. Hierzu wird zunächst ein Zeitintervall T_{win} bestimmt, das vor der zu dem relativen Maximum gehörigen Oszillation Oₙ liegt. Das Zeitfenster T_{win} wird in Abhängigkeit der Pulsfrequenz bestimmt, insbesondere als Bruchteil des Zeitintervalls Tₚᵤₗₛ, das zwischen zwei aufeinanderfolgenden Oszillationen liegt. Zur Bestimmung des Zeitintervalls Tₚᵤₗₛ wird die Differenz zwischen den Zeitpunkten der Oszillationsmaxima der beiden entsprechenden aufeinanderfolgenden Oszillationen in einem Verfahrensschritt bestimmt. Vorzugsweise beträgt die Breite des Zeitintervalls T_{win} etwa ein Viertel des Oszillations-Zeitabstandes Tₚᵤₗₛ.

In einem nächsten Verfahrensschritt wird die Lage des Zeitfensters T_{win} relativ zu dem Maximum der Oszillation Oₙ bestimmt, und zwar in Abhangigkeit der Pulsfrequenz, insbesondere als Bruchteil des Zeitintervalls Tₚᵤₗₛ. Das Zeitfenster T_{win} wird dabei um einen Abstand d vor den Zeitpunkt des Oszillationsmaximums der Oszillation Oₙ gelegt. der derart bemessen ist, daß das Zeitintervall T_{win} etwa an dem Punkt B endet, welcher dem Anfang der Oszillation Oₙ entspricht (vgl. Figur 3B). Vorzugsweise kann der Abstand d etwa 1/4 Tₚᵤₗₛ betragen. Das Zeitfenster T_{win} ist also derart bestimmt, daß es auf einen Bereich begrenzt ist, in dem die Manschettendruckwerte nicht von einer Oszillation überlagert sind und das Zeitfenster T_{win} vorzugsweise möglichst nahe vor einer Oszillation liegt.

In einem weiteren Verfahrensschritt wird dann mittels einer Mittelwerts-Bestimmungseinrichtung 15 der Mittelwert Pₘₙ der in dem Zeitfenster T_{win} erfaßten Manschettendruckwerte bestimmt und ein Zeitwert tₘₙ zugeordnet, der durch die Mitte des Zeitfensters T_{win} bestimmt ist.

Analog zu der Bestimmung des Druckmittelwertes Pₘₙ zum Zeitpunkt tₘₙ wird in einem weiteren Verfahrensschritt ein vor der vorhergehenden Oszillation Oₙ₋₁ liegender Druckmittelwert Pₘₙ₋₁ zum Zeitpunkt tₘₙ₋₁ bestimmt. Mittels einer Interpolationseinrichtung 16 wird dann zwischen den Druckmittelwerten Pₘₙ₋₁ und Pₘₙ linear interpoliert und der Fußpunkt Fₙ₋₁ zum Zeitpunkt tₙ₋₁, an dem das Maximum der Oszillation Oₙ₋₁ aufgetreten ist, bestimmt. Der Fußpunkt Fₙ₋₁ wird als Schnittpunkt der Geraden durch die Druckmittelwerte Pₘₙ₋₁ und Pₘₙ und der Senkrechten durch das Oszillationsmaximum Mₙ₋₁ der Oszillation Oₙ₋₁ bestimmt. Der Fußpunkt Fₙ₋₁ entspricht dem wahren Manschettendruck zum Zeitpunkt tₙ₋₁. Die Differenz zwischen dem Oszillationsmaximum Mₙ₋₁ und dem Fußpunkt Fₙ₋₁ entspricht der Amplitude der Oszillation Oₙ₋₁.

Diese oben geschilderte Art der Fußpunktbestimmung in der Hauptsache mittels einer Interpolationseinrichtung setzt jedoch voraus, daß der Luftdruck in der Manschette 1 z.B. durch ein Regelventil kontinuierlich veränderbar bzw. verminderbar ist. In einer alternativen Ausführungsform wird die Fußpunktbestimmung in der Hauptsache mit einer Extrapolationseinrichtung als Approximationseinrichtung für die stufenweise Veränderung oder Verminderung des Luftdruckes in der Manschette z.B. durch ein nicht dargestelltes Schaltventil durchgeführt. Hierzu wird eine Gerade durch die Druckmeßwerte in einem Mittelungsfenster im Zeitinervall T_{win} zunächst linear interpoliert (Bildung einer Regressionsgerade) Das Mittelungsfenster liegt also hier einseitig vor der jeweiligen Oszillation Die so ermittelte Gerade entspricht etwa dem konstanten Druck, der fur eine bestimmte Zeit beim stufenweisen Luftablassen sozusagen ein Druckplateau in der Druckablaßkurve bildet. Der Fußpunkt wird dann durch eine Verlängerung bzw. Extrapolation dieser Gerade zur zugehörigen nachfolgenden Oszillation und dem Schnittpunkt Fₙ der verlängerten Gerade mit der Vertikalen am Oszillationsmaximum Mₙ gebildet. Es wird also in diesem Fall der Fußpunkt Fₙ und nicht Fₙ₋₁ bestimmt.

In einer weiteren Variante sind Mischformen beider Fußpunktbestimmungsverfahren vorgesehen.

Jede der Oszillationen wird durch das Oszillationsmaximum, den zugehörigen Fußpunkt und den entsprechenden Zeitpunkt, an dem das Oszillationsmaximum aufgetreten ist, ausreichend gekennzeichnet. Mittels einer Datenreduktionseinrichtung 17 werden diese drei die Oszillation kennzeichnenden Werte im Speicher 8 abgelegt, während die übrigen von der Druckerfassungseinrichtung 6 im Bereich der jeweiligen Oszillation erfaßten Druckmeßwerte gelöscht werden können. Die Fußpunktbestimmung und die damit verbundene Datenreduktion wird parallel zum Meßvorgang während des Druckablaßvorganges bewirkt, so daß die im Speicher 8 gespeicherte Datenmenge beträchtlich reduziert werden kann. Der wahre Manschettendruck ist durch die Fußpunkte und gegebenenfalls die vorher bestimmten Druckmittelwerte bestimmt. Die frühzeitige Datenreduktion bewirkt neben der erleichterten Datenspeicherung auch eine erhebliche Verminderung des notwendigen Rechenaufwands.

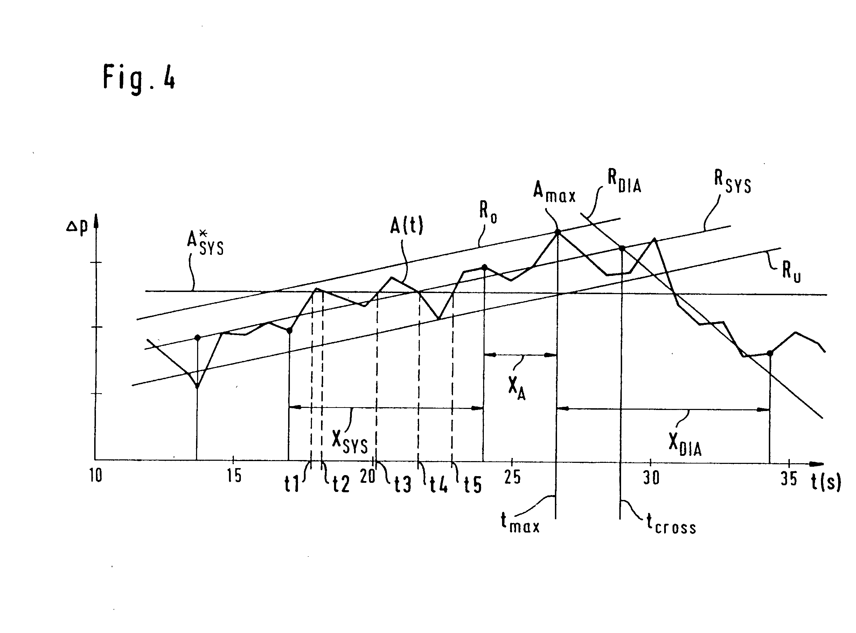

Zur weiteren Berechnung der Blutdruckwerte wird mittels einer Maximum-Berechnungseinrichtung 18 das Auftreten einer wahren maximalen Oszillation Oₘₐₚ aus den Amplituden tatsächlich erfaßter Oszillationen berechnet. Hierzu wird in einem ersten Schritt zunächst aus den tatsächlich erfaßten Oszillationen die Oszillation bestimmt, deren Amplitude Aₘₐₓ maximal ist (vgl. Figur 4) Das Auftreten der wahren maximalen Oszillation Oₘₐₚ wird in weiteren Verfahrensschritten in Abhangigkeit von zwei linearen Regressionen bestimmt, von denen eine durch die Amplituden von Oszillationen, die vor der erfaßten maximalen Oszillation erfaßt wurden, und die andere durch die Amplituden von Oszillationen. die nach der erfaßten maximalen Oszillation erfaßt wurden, bestimmt werden. Die maximale Oszillation selbst wird insbesondere also fur die Regressionsgeraden nicht verwendet bzw nur hinsichtlich ihres Zeitpunktes.

Mittels einer Regressionseinrichtung 19 wird eine Regressionsgerade durch die Amplitudenwerte von in der Amplitude ansteigenden Oszillationen, das heißt vor der maximalen Oszillation erfaßten Oszillationen, die nachfolgend systolische Regressionsgerade genannt wird, und eine zweite Regressionsgerade durch die Amplituden von in der Amplitude kleiner werdenden Oszillationen, das heißt nach der maximalen Oszillation erfaßten Oszillationen, die nachfolgend diastolische Regressionsgerade genannt wird, bestimmt. Für die Bestimmung der Regressionsgeraden werden jeweils mittels einer Auswahleinrichtung 20 die Amplituden bestimmter Oszillationen ausgewählt, die im Bereich eines systolischen bzw. diastolischen Erwartungswertes erfaßt wurden.

Zur Bestimmung der systolischen Regressionsgeraden R_{SYS} wird zunächst ein Zeitintervall X_{SYS} festgelegt, innerhalb dessen die Oszillationen erfaßt wurden, deren Amplituden für die Regressionsgerade R_{SYS} verwendet werden. Die Länge des Zeitfensters X_{SYS} wird in Abhängigkeit der Herzfrequenz bestimmt, vorzugsweise derart, daß zumindest fünf Herzschläge, insbesonders 5 bis 9 Herzschläge und die entsprechende Anzahl von Oszillationen berücksichtigt werden. Die Herzfrequenz bzw. das zwischen zwei Pulsschlägen liegende Zeitintervall Tₚᵤₗₛ wurde bereits zuvor bei der Bestimmung des wahren Manschettendrucks berechnet. Die Mitte des Zeitfensters X_{SYS} wird in Abhängigkeit eines systolischen Erwartungswertes A*_{SYS} berechnet. Der systolische Erwartungswert ist gerätespezifisch und wird als Bruchteil der tatsächlich erfaßten maximalen Amplitude Aₘₐₓ bestimmt. Der systolische Erwartungswert ist dadurch bestimmt, daß bei dem Manschettendruckwert, der dem systolischen Blutdruck entspricht, eine Oszillation auftritt, deren Amplitude einem bestimmten Bruchteil der Amplitude der maximalen Oszillation, die bei dem mittleren arteriellen Blutdruck auftritt, entspricht. Dieses sogenannte Systolenverhältnis kann 0,2 bis 0,8, bei einer kleinen Handgelenksmanschette beispielsweise etwa 0.78, betragen. Zur Bestimmung des Mittelpunktes des Zeitfensters X_{SYS} wird der systolische Erwartungswert A*_{SYS} (vgl. Figur 4) mit dem zeitlichen Verlauf der erfaßten Amplituden A(t) gleichgesetzt, das heißt die Gerade A*_{SYS} wird mit dem Graphen A(t) geschnitten. Da der zeitliche Verlauf der erfaßten Amplituden A(t) typischerweise nicht monoton ist (vgl. Figur 4), werden auf diese Weise mehrere Schnittpunkte und damit mehrere Zeitpunkte t1 bis t5 (vgl Figur 4) bestimmt Als Mitte des Zeitintervalls X_{SYS} wird der Mittelwert der auf diese Weise bestimmten Zeitpunkte t1 bis t5 bestimmt. Das Zeitintervall X_{SYS} liegt somit symmetrisch um diesen Mittelwert und besitzt die oben beschriebene herzfrequenzabhangige Breite. Falls die Breite des Zeitintervalls X_{SYS} derart ist, daß ein Abschnitt des Zeitfensters X_{SYS} den systolischen Bereich überschreitet, das heißt der entsprechende Abschnitt in Figur 4 rechts vom Zeitpunkt tₘₐₓ liegt, wird der betroffene Teil des Zeitintervalls X_{SYS} entsprechend verkürzt. Zwischen dem Zeitintervall X_{SYS} und dem Auftreten der maximalen Amplitude Aₘₐₓ, dem Zeitpunkt tₘₐₓ, liegt also ein zeitlicher Abstand X_{A}, der kleinstenfalls 0 ist (vgl. Figur 4).

Von der Auswahleinrichtung 20 werden die innerhalb des Zeitintervalls X_{SYS} liegenden Amplitudenwerte ausgewählt und die Regressionseinrichtung 19 bildet dann in Abhängigkeit der ausgewählten Amplitudenwerte die Regressionsgerade R_{SYS}.

In einem nächsten Verfahrensschritt wird eine Fehlerdämpfung bewirkt. Hierzu werden um die systolische Regressionsgerade R_{SYS} zwei parallele Grenzgeraden R_{O} und R_{U} bestimmt, die parallel zur Regressionsgeraden R_{SYS} liegen und deren Abstand zur Regressionsgeraden R_{SYS} der x-fachen Standardabweichung (3s) Wertedifferenzen (=Meßwert minus Rechenwert) im Berechnungsfenster entspricht (vgl. Figur 4). Der Wert x wurde vorteilhaft mit x=2 bis x=3 festgelegt. In einem weiteren Schritt werden die erfaßten Amplitudenwerte mit den beiden Grenzgeraden R_{O} und R_{U} verglichen. Liegt einer der Amplitudenwerte außerhalb dieser Grenzen, muß von einem Artefakt ausgegangen werden. Zur Fehlerbegrenzung wird der entsprechende Amplitudenwert durch den jeweiligen Wert der Regressionsgerade R_{SYS} zu dem entsprechenden Zeitpunkt ersetzt. Nach dieser Signalfilterung wird die Regressionsgerade R_{SYS} erneut mit den gegebenenfalls ersetzten Werten im Berechnungsfenster X_{SYS} berechnet.

Analog zur beschriebenen Bestimmung der systolischen Regressionsgeraden R_{SYS} wird dann von der Regressionseinrichtung 19 die diastolische Regressionsgerade R_{DIA} (vgl. Figur 4) bestimmt. Die beiden Grenzgeraden R_{o'} und R_{u'} , die parallel zu R_{DIA} verlaufen und die x-fache Standardabweichung zur diastolischen Regressionsgeraden festlegen und analog zur systolischen Seite, wie oben beschrieben, verwendet werden, sind in Fig. 4 nicht gezeigt. Abweichend wird jedoch die charakteristische Lage und Breite des Zeitfensters X_{DIA} bestimmt. Die Breite des Zeitintervalls wird ebenfalls in Abhängigkeit der Herzfrequenz bestimmt, jedoch werden vorzugsweise zumindest 4, insbesondere 4 bis 6 Herzschlage bzw entsprechende Oszillationen berücksichtigt. Die Lage des Zeitintervalls X_{DIA} wird derart festgelegt, daß das Zeitfenster X_{DIA} unmittelbar am Zeitpunkt tₘₐₓ der erfaßten maximalen Oszillation Aₘₐₓ beginnt (vgl. Figur 4).

In einem weiteren Verfahrensschritt wird mittels einer Schnittpunkt-Bestimmungseinrichtung 22 der Schnittpunkt der beiden Regressionsgeraden R_{SYS} und R_{DIA} bestimmt. Der Zeitpunkt tₘₐₚ, an dem die wahre maximale Oszillation auftritt, wird in Abhängigkeit des durch den Schnittpunkt der beiden Regressionsgeraden bestimmten Zeitpunkts t_{cross} und dem Zeitpunkt tₘₐₓ der tatsächlich erfaßten maximalen Amplitude bestimmt, insbesondere mittels einer Mittelwert-Bestimmungseinrichtung 21 als Mittelwert ( oder alternativ in einem anderen Verhältnis) der beiden Zeitpunkte t_{cross} und tₘₐₓ bestimmt.

In einem weiteren Verfahrensschritt wird von einer Blutdruckwerte-Bestimmungseinrichtung 23 zunächst der mittlere arterielle Blutdruck MAP bestimmt. Hierzu wird festgestellt, welcher Manschettendruckwert P zum Zeitpunkt tₘₐₚ der wahren maximalen Oszillation erfaßt wurde. Die wahren Manschettendruckwerte sind zusammen mit den zugehörigen Erfassungszeitpunkten in dem Speicher 8 abgelegt bzw können gegebenenfalls durch Interpolation aus diesen bestimmt werden.

Zur Bestimmung des systolischen Blutdruckwerts wird in einem weiteren Verfahrensschritt der Zeitpunkt bestimmt, zu dem die den systolischen Blutdruck kennzeichnende Oszillation aufgetreten ist. Diese Oszillation ist durch eine Amplitude bestimmter Höhe gekennzeichnet, die einen bestimmten Bruchteil der maximalen Amplitude bildet und gerätespezifisch festliegt. Der Zeitpunkt, zu dem die den systolischen Blutdruckwert kennzeichnende Amplitude auftritt, wird mit Hilfe der Regressionsgeraden R_{SYS} bestimmt. Hierzu wird berechnet, zu welchem Zeitpunkt die Regressionsgerade R_{SYS} den als Bruchteil der maximalen Amplitude bestimmten systolischen Amplitudenwert aufweist Die Regressionsgerade R_{SYS} dient also neben der Bestimmung des Zeitpunktes tₘₐₚ auch der Bestimmung des Zeitpunktes der systolischen Amplitude. Dies vermindert den notwendigen Rechenaufwand beträchtlich.

Mit Hilfe der Blutdruckwerte-Bestimmungseinrichtung 23 wird dann der wahre Manschettendruckwert zu dem entsprechenden Zeitpunkt bestimmt In analoger Weise wird der diastolische Blutdruckwert bestimmt, wobei hier der Abstand X_{A} des Berechnungsfensters X_{DIA} typischerweise zu Null wird aufgrund der steileren Steigung der diastolischen Regressionsgeraden R_{DIA} im Vergleich zur Systolischen.

Gemäß einer vorteilhaften Ausführung der Erfindung können die Funktionen der Einrichtungen 12 bis 23 der Auswerteeinheit 10 durch entsprechende Programmierung eines Mikrokontrollers bewirkt werden.

Die vorliegende Erfindung erlaubt eine rasche Bestimmung der Blutdruckwerte, die darüber hinaus eine Bestimmung und Beseitigung von Störungen, die dem Druckmeßsignal überlagert sind und besonders bei Handgelenksgeräten häufig auftreten, erlaubt. Insbesondere die frühe Datenreduktion auf drei Meßwerte pro Pulsschlag, nämlich das Oszillationsmaximum, der berechnete Oszillationsfußpunkt und der Zeitpunkt des Pulsschlags während der Messung bzw. Druckerfassung erlaubt eine rasche Auswertung, die mit geringer Speichertiefe auskommt.

Durch die Frequenzanpassung der Zeitintervalle, aus denen Meßwerte zur Auswertung ausgewählt werden, kann bei Erreichen des unteren diastolischen Blutdrucks die Manschette entlüftet werden. Damit wird die individuelle Meßzeit erheblich reduziert, der Patient wird schmerzentlastet, so daß er die Blutdruckmessung konzentrierter und entspannter ausführen kann und dementsprechend störende Bewegungsartefakte, bedingt durch Befreiungsbewegungen vermieden werden können.

## Patentansprüche

1. Verfahren zur Bestimmung des Blutdrucks, wobei mittels einer Manschette ein Körperglied abgeschnürt wird, ein Manschettendruck verändert und mittels einer Druckerfassungseinrichtung erfaßt wird, Amplituden von erfaßten Oszillationen des Manschettendrucks bestimmt werden, aus den Amplituden erfaßter Oszillationen das Auftreten einer wahren maximalen Oszillation bestimmt wird und Blutdruckwerte in Abhängigkeit von der wahren maximalen Oszillation bestimmt werden, wobei zwei lineare Regressionen (R_{SYS}, R_{DIA}) bestimmt werden, von denen eine durch die Amplituden von Oszillationen, die vor der erfaßten maximalen Oszillation (Aₘₐₓ) erfaßt wurden, und die andere durch die Amplituden von Oszillationen, die nach der erfaßten maximalen Oszillation (Aₘₐₓ) erfaßt wurden, bestimmt werden, **dadurch gekennzeichnet, daß** das Auftreten der wahren maximalen Oszillation zum einen in Abhängigkeit eines Schnittpunktes (tcross) der zwei linearen Regressionen (R_{SYS}, R_{DIA}) und zum anderen in Abhängigkeit von dem Auftreten der erfaßten maximalen Oszillation bestimmt wird, wobei das Auftreten der wahren maximalen Oszillation aus einem Verhältniswert des Schnittpunktes (tcross) und dem Auftreten der erfaßten maximalen Oszillation berechnet wird.

2. Verfahren nach Anspruch 1, **dadurch gekennzeichnet, daß** als Verhältniswert der Mittelwert des Schnittpunktes (tcross) und dem Auftreten der erfaßten maximalen Oszillation berechnet wird

3. Verfahren nach Anspruch 1 oder 2, **dadurch gekennzeichnet, daß** ein wahrer Manschettendruck, der frei von Oszillationen ist. bestimmt wird und die Blutdruckwerte aus dem wahren Manschettendruck bestimmt werden.

4. Verfahren nach zumindest einem der vornergehenden Ansprüche, **dadurch gekennzeichnet, daß** vor und / oder nach einer Oszillation erfaßte Manschettendruckwerte (P) in den Bereich der Oszillation approximiert werden.

5. Verfahren nach zumindest einem der vorhergehenden Ansprüche, **dadurch gekennzeichnet, daß** für jede Oszillation (Oₙ) ein zu einem jeweiligen Oszillationsmaximum (Mₙ) zugehöriger Fußpunkt (Fₙ) bestimmt wird und in einer Speichereinrichtung (8) ein Datendreisatz abgespeichert wird, der das jeweilige Oszillationsmaximum, den zugehörigen Fußpunkt und einen zugehörigen Zeitpunkt (tₙ) an dem das Oszillationsmaximum erfaßt wurde, aufweist.

6. Verfahren nach zumindest einem der vorhergehenden Ansprüche, **dadurch gekennzeichnet, daß** von Manschettendruckwerten (P) vor und nach einer Oszillation (Oₙ) jeweils Mittelwerte (Pₘₙ) bestimmt werden und in Abhängigkeit der jeweiligen Mittelwerte der zugehörige Fußpunkt (Fₙ) bestimmt wird.

7. Verfahren nach zumindest einem der vorhergehenden Ansprüche, **dadurch gekennzeichnet, daß** zur Bestimmung der Regressionsgeraden (R_{SYS}, R_{DIA}) Oszillationen, die in einem begrenzten Bereich (X_{SYS}, Y_{DIA}) um einen jeweiligen systolischen und diastolischen Erwartungswert (A*) erfaßt wurden, ausgewählt werden, wobei vorzugsweise der begrenzte Bereich in Abhängigkeit der Herzfrequenz bestimmt wird.

8. Verfahren nach zumindest einem der vorhergehenden Ansprüche, **dadurch gekennzeichnet, daß** ein Auftreten einer Oszillation, das einen systolischen bzw. einen diastolischen Blutdruckwert kennzeichnet, aus den beiden Regressionsgeraden (R_{SYS}, R_{DIA}) bestimmt wird.

9. Blutdruckmeßgerät mit einer Manschette (1) zum Abschnüren eines Körperglieds, einer Drucksteuereinrichtung (5) zur Steuerung eines Manschettendrucks, einer Druckerfassungseinrichtung (6) zur Erfassung des Manschettendrucks, einer Amplituden-Bestimmungseinrichtung (13, 14) zur Bestimmung der Amplituden von Oszillationen des Manschettendrucks, einer Maximum-Berechnungseinrichtung (18) zur Berechnung des Auftretens einer wahren maximalen Oszillation aus den Amplituden erfaßter Oszillationen und einer Blutdruckwerte-Berechnungseinrichtung (23) zur Berechnung von Blutdruckwerten in Abhängigkeit der wahren maximalen Oszillation, wobei zwei Regressionsgeraden bestimmbar sind, von denen eine durch die Amplituden von Oszillationen, die vor der erfaßten maximalen Oszillation erfaßt wurden, und die andere durch die Amplituden von Oszillationen, die nach der erfaßten maximalen Oszillation erfaßt wurden, bestimmt wird, **dadurch gekennzeichnet, daß** die Maximum-Berechnungseinrichtung (18) eine Einrichtung (19, 21, 22) aufweist, die das Auftreten der wahren maximalen Oszillation einerseits in Abhangigkeit vom Schnittpunkt (tcross) der zwei Regressionsgeraden (R_{SYS}, R_{DIA}) und andererseits in Abhängigkeit der erfaßten maximalen Oszillation bestimmt, wobei die wahre maximale Oszillation als Verhältniswert aus dem Schnittpunkt und der erfaßten maximalen Oszillation berechenbar ist.

10. Blutdruckmeßgerät nach Anspruch 9, **dadurch gekennzeichnet, daß** die Einrichtung, die das Auftreten der wahren maximalen Oszillation bestimmt, derart ausgebildet ist, daß als Verhältniswert der Mittelwert des Schnittpunkts und des Auftretens der erfaßten maximalen Oszillation berechenbar ist.

11. Blutdruckmeßgerät nach Anspruch 9 oder 10, **dadurch gekennzeichnet, daß** eine Oszillations-Filtereinrichtung (12) zur Bestimmung eines wahren Manschettendrucks frei von Oszillationen (Oₙ) vorgesehen ist und die Blutdruckwerte-Berechnungseinrichtung (23) derart ausgebildet ist, daß die Blutdruckwerte aus dem wahren Manschettendruck bestimmt werden.

12. Blutdruckmeßgerät nach zumindest einem der Ansprüche 9 bis 11, **dadurch gekennzeichnet, daß** die Oszillations-Filtereinrichtung (12) eine Approximationseinrichtung, vorzugsweise eine Interpolationseinrichtung (16) zur Interpolation von vor und nach einer Oszillation (Oₙ) erfaßten Manschettendruckwerte in den Bereich der Oszillation aufweist.

13. Blutdruckmeßgerät nach zumindest einem der Ansprüche 9 bis 12, **dadurch gekennzeichnet, daß** eine Datenreduktionseinrichtung (17) zur Reduktion von Daten des erfaßten Manschettendrucks (P) vorgesehen ist, wobei vorzugsweise die Datenreduktionseinrichtung eine Fußpunkt-Bestimmungseinrichtung (14) zur Bestimmung eines zu einem jeweiligen Oszillationsmaximum (Mₙ) zugehörigen Fußpunkts (Fₙ) aufweist und eine Speichereinrichtung (8) zur Abspeicherung des jeweiligen Oszillationsmaximums, des zugehörigen Fußpunktes und des Zeitpunktes (tₙ), an dem das Oszillationsmaximum erfaßt wurde, als Datensatz vorgesehen ist.

14. Blutdruckmeßgerät nach Anspruch 13, **dadurch gekennzeichnet, daß** die Fußpunkt-Bestimmungseinrichtung (14) eine Mittelwert-Bestimmungseinrichtung (21) zur Bestimmung von Mittelwerten (Pₘ) von vor und nach einer Oszillation (Oₙ) erfaßten Manschettendruckwerten und eine Berechnungseinheit (16) zur Berechnung des jeweiligen Fußpunkts (Fₙ) in Abhängigkeit der Mittelwerte aufweist.

15. Blutdruckmeßgerät nach zumindest einem der Ansprüche 9 bis 14, **dadurch gekennzeichnet, daß** die Maximum-Berechnungseinrichtung (18) eine Auswahleinrichtung (20) zur Auswahl von Oszillationen zur Bestimmung der Regressionsgeraden (R_{SYS}, R_{DIA}) aufweist, wobei vorzugsweise die Auswahleinrichtung eine Erwartungswert-Berechnungseinrichtung zur Berechnung eines systolischen und diastolischen Erwartungswerts (A*) und eine Bereichs-Berechnungseinrichtung zur Berechnung je eines Bereichs (X_{SYS}, X_{DIA}) um den systolischen und diastolischen Erwartungswert insbesondere in Abhängigkeit der Herzfrequenz aufweist.

16. Blutdruckmeßgerät nach zumindest einem der Ansprüche 9 bis 15, **dadurch gekennzeichnet, daß** die Blutdruckwerte-Berechnungseinrichtung (23) eine Einrichtung aufweist, die ein Auftreten einer Oszillation, das einen systolischen bzw. einen diastolischen Blutdruckwert kennzeichnet, aus den beiden Regressionsgeraden (R_{SYS}, R_{DIA}) bestimmt.

## Claims

1. A method for measuring the blood pressure, comprising the steps of compressing a subject's limb by means of an occlusive cuff, modifying and detecting a cuff pressure by means of a pressure sensing device, determining amplitudes of detected cuff pressure oscillations, determining the presence of a true maximum oscillation from the amplitudes of detected oscillations, and determining blood pressure values in dependence upon the true maximum oscillation, there being determined two linear regressions (R_{SYS}, R_{DIA}), one from the amplitudes of oscillations detected before the maximum oscillation (Aₘₐₓ) was detected, and the other from the amplitudes of oscillations detected after the maximum oscillation (Aₘₐₓ) was detected, **characterized in that** the presence of the true maximum oscillation is determined on the one hand in dependence upon a point of intersection (t_{cross}) of the two linear regressions (R_{SYS}, R_{DIA}), and on the other hand in dependence upon the occurrence of the detected maximum oscillation, the occurrence of the true maximum oscillation being computed from a ratio of the point of intersection (t_{cross}) to the occurrence of the detected maximum oscillation.

2. The method as claimed in claim 1, **characterized in that** as ratio the mean value of the point of intersection (t_{cross}) to the occurrence of the detected maximum oscillation is calculated.

3. The method as claimed in claim 1 or 2, **characterized in that** a true cuff pressure devoid of oscillations is determined, and that the blood pressure values are determined from the true cuff pressure.

4. The method as claimed in at least one of the preceding claims, **characterized in that** cuff pressure values (P) detected prior and/or subsequent to an oscillation are approximated in the range of oscillation.

5. The method as claimed in at least one of the preceding claims, **characterized in that** for each oscillation (Oₙ) a foot point (Fₙ) associated with a respective maximum in oscillation (Mₙ) is determined, and that in a storage unit (8) a data triplet is stored containing the respective maximum in oscillation, the associated foot point and an associated instant of time (tₙ) at which the maximum in oscillation was detected.

6. The method as claimed in at least one of the preceding claims, **characterized in that** respective mean values (Pₘₙ) of cuff pressure values (P) are determined prior and subsequent to an oscillation (Oₙ), and the associated foot point (Fₙ) is determined in dependence upon the respective mean values.

7. The method as claimed in at least one of the preceding claims, **characterized in that** for determination of the regression lines (R_{SYS}, R_{DIA}), oscillations are selected which were detected in a limited range (X_{SYS}, Y_{DIA}) about a respective systolic and diastolic expected value (A*), with preferably the limited range being determined in dependence upon the heart frequency.

8. The method as claimed in at least one of the preceding claims, **characterized in that** the occurrence of an oscillation characteristic of a systolic or diastolic pressure value is determined from the two regression lines (R_{SYS}, R_{DIA}).

9. A blood pressure measuring device having an occlusive cuff (1) for compressing a subject's limb, a pressure control device (5) for controlling a cuff pressure, a pressure sensing device (6) for detecting the cuff pressure, an amplitude determining device (13, 14) for determining the amplitudes of cuff pressure oscillations, a maximum value computing unit (18) for computing the occurrence of a true maximum oscillation from the amplitudes of detected oscillations, and a blood pressure value computing device (23) for computing blood pressure values in dependence upon the true maximum oscillation, wherein two regression lines are determinable, one of which is determined from the amplitudes of oscillations detected before the maximum oscillation was detected, while the other is determined from the amplitudes of oscillations detected after the maximum oscillation was detected, **characterized in that** the maximum value computing unit (18) comprises a device (19, 21, 22) determining the presence of the true maximum oscillation on the one hand in dependence upon the point of intersection (t_{cross}) of the two regression lines (R_{SYS}, R_{DIA}), and on the other hand in dependence upon the detected maximum oscillation, with the true maximum oscillation being computable as a ratio of the point of intersection to the detected maximum oscillation.

10. The blood pressure measuring device as claimed in claim 9, **characterized in that** the device determining the occurrence of the true maximum oscillation is configured such that as ratio the mean value of the point of intersection to the occurrence of the detected maximum oscillation is computable.

11. The blood pressure measuring device as claimed in claim 9 or 10, **characterized in that** provision is made for an oscillation filtering device (12) for determining a true cuff pressure devoid of oscillations (Oₙ), and that the blood pressure value computing device (23) is configured such that the blood pressure values are determined from the true cuff pressure.

12. The blood pressure measuring device as claimed in at least one of the claims 9 to 11, **characterized in that** the oscillation filtering device (12) comprises an approximation device, preferably an interpolating device (16) for the interpolation of cuff pressure values detected prior and subsequent to an oscillation (On) in the range of the oscillation.

13. The blood pressure measuring device as claimed in at least one of the claims 9 to 12, **characterized in that** provision is made for a data reduction device (17) for reducing detected cuff pressure data (P), said data reduction device comprising preferably a foot point determining device (14) for determining a foot point (Fₙ) associated with a respective maximum in oscillation (Mₙ), and that a storage unit (8) is provided for storing as a data record the respective maximum in oscillation, the associated foot point and the instant of time (tₙ) at which the maximum in oscillation was detected.

14. The blood pressure measuring device as claimed in claim 13, **characterized in that** the foot point determining device (14) comprises a mean value determining device (21) for the determination of mean values (Pₘ) of cuff pressure values detected prior and subsequent to an oscillation (Oₙ), and a computing unit (16) for computing the respective foot point (Fₙ) in dependence upon the mean values.

15. The blood pressure measuring device as claimed in at least one of the claims 9 to 14, **characterized in that** the maximum value computing unit (18) includes a selection device (20) for the selection of oscillations for determining the regression lines (R_{SYS}, R_{DIA}), said selection device having preferably an expected value computing device for computing a systolic and a diastolic expected value (A*), and a range computing device for computing a respective range (X_{SYS}, X_{DIA}) about the systolic and the diastolic expected values, in particular in dependence upon the heart frequency.

16. The blood pressure measuring device as claimed in at least one of the claims 9 to 15, **characterized in that** the blood pressure value computing device (23) possesses a device determining the occurrence of an oscillation characteristic of a systolic or diastolic blood pressure value from the two regression lines (R_{SYS}, R_{DIA}).

## Revendications

1. Procédé pour déterminer la pression sanguine, selon lequel à l'aide d'un manchon on comprime une partie du corps, on modifie la pression du manchon et on effectue une détection à l'aide du dispositif de détection de pression, on détermine des amplitudes d'oscillations détectées de la pression du manchon, à partir des amplitudes d'oscillations détectées on détermine l'apparition d'une oscillation maximale réelle et on détermine les valeurs de la pression sanguine en fonction de l'oscillation maximale vraie, et selon lequel on détermine deux régressions linéaires (R_{SYS}, R_{DIA}), dont l'une est déterminée par les amplitudes d'oscillations qui ont été détectées avant l'oscillation maximale détectée (Aₘₐₓ), et dont l'autre est déterminée au moyen des amplitudes d'oscillations, qui ont été détectées après l'oscillation maximale détectée (Aₘₐₓ), **caractérisé en ce que** l'apparition de l'oscillation maximale vraie est déterminée d'une part en fonction d'un centre d'intersection (tcross) des deux régressions linéaires (R_{SYS}, R_{DIA}), et d'autre part en fonction de l'apparition de l'oscillation maximale détectée, l'apparition de l'oscillation maximale vraie étant calculée à partir d'une valeur de rapport du point d'intersection (tcross) et de l'apparition de l'oscillation maximale détectée.

2. Procédé selon la revendication 1, **caractérisé en ce que** l'on calcule en tant que valeur de rapport la valeur moyenne du point d'intersection (tcross) et l'apparition de l'oscillation maximale détectée.

3. Procédé selon la revendication 1 ou 2, **caractérisé en ce qu'**on détermine une pression vraie du manchon, qui est exempte d'oscillations, et qu'on détermine les valeurs de la pression sanguine à partir de la pression vraie du manchon.

4. Procédé selon au moins l'une des revendications précédentes, **caractérisé en ce qu'**on approxime les valeurs de pression du manchon (P) détectées avant et/ou après une oscillation, dans la zone de l'oscillation.

5. Procédé selon au moins l'une des revendications précédentes, **caractérisé en ce que** pour chaque oscillation (Oₙ), on détermine un point de base (Fₙ), qui est associé au maximum respectif d'oscillation (On), et qu'on mémorise dans un dispositif de mémoire (8) un triplet de données qui possède le maximum respectif d'oscillation, le point de base associé et un instant associé (tₙ), lors duquel le maximum de l'oscillation a été détecté.

6. Procédé selon au moins l'une des revendications précédentes, **caractérisé en ce qu'**on détermine respectivement des valeurs moyennes (Pₘₙ) des valeurs (P) de la pression du manchon avant et après une oscillation (Oₙ) et qu'on détermine la valeur de base associée (Fₙ) en fonction des valeurs moyennes respectives.

7. Procédé selon au moins l'une des revendications précédentes, **caractérisé en ce que** pour déterminer les droites de régression (R_{SYS}, R_{DIA}), on sélectionne des oscillations, qui ont été détectées dans une zone limitée (Y_{SYS}, Y_{DIA}) autour des valeurs respectives d'espérance systolique et diastolique (A*), auquel cas on détermine de préférence la zone limitée en fonction de la fréquence cardiaque.

8. Procédé selon au moins l'une des revendications précédentes, **caractérisé en ce qu'**une apparition d'une oscillation, qui caractérise une valeur systolique ou une valeur diastolique de la pression sanguine, est déterminée à partir des deux droites de régression (R_{SYS}, R_{DIA}).

9. Appareil de mesure de la pression sanguine comportant un manchon (1) servant à étrangler un organe corporel, un dispositif de commande de pression (5) pour commander une pression du manchon, un dispositif (6) de détection de la pression pour détecter la pression du manchon, un dispositif de détermination d'amplitude (13, 14) pour déterminer les amplitudes d'oscillations de la pression du manchon, un dispositif de calcul de maximum (18) pour calculer l'apparition d'une oscillation maximale vraie à partir des amplitudes d'oscillation détectées et un dispositif (23) de calcul de valeurs de la pression sanguine pour calculer des valeurs de la pression sanguine en fonction de l'oscillation maximale vraie, dans lequel on peut déterminer deux droites de régression, dont l'une est déterminée par les amplitudes d'oscillation, qui ont été détectées avant l'oscillation maximale détectée, et dont l'autre est déterminée par les amplitudes d'oscillation, qui ont été détectées après l'oscillation maximale détectée, **caractérisé en ce que** le dispositif (18) de calcul du maximum comporte un dispositif (19, 21, 22) qui détermine l'apparition de l'oscillation maximale vraie, en fonction du point d'intersection (tcross) des deux droites de régression (R_{SYS}, R_{DIA}) et d'autre part en fonction de l'oscillation maximale détectée, l'oscillation maximale vraie pouvant être calculée en tant que valeur de rapport à partir du point d'intersection et de l'oscillation maximale détectée.

10. Appareil de mesure de la pression sanguine selon la revendication 9, **caractérisé en ce que** le dispositif, qui détermine l'apparition d'oscillations maximales vraies, est agencé de telle sorte qu'en tant que valeur de rapport, on peut calculer la valeur moyenne du point d'intersection et de l'apparition de l'oscillation maximale détectée.

11. Appareil de mesure de la pression sanguine selon la revendication 9 ou 10, **caractérisé en ce qu'**il est prévu un dispositif de filtrage d'oscillations (12) pour déterminer une pression de manchon, exempte d'oscillations (Oₙ), et le dispositif (23) de calcul de la valeur de pression sanguine est agencé de telle sorte que les valeurs de la pression sanguine sont déterminées à partir de la pression vraie du manchon.

12. Appareil de mesure de la pression sanguine selon au moins l'une des revendications 1 à 11, **caractérisé en ce que** le dispositif (12) de filtrage d'oscillations possède un dispositif d'approximation, de préférence un dispositif d'interpolation (16) pour interpoler des valeurs de la pression du manchon détectées avant et après une oscillation (On) dans la zone de l'oscillation.

13. Appareil de mesure de la pression sanguine selon au moins l'une des revendications 9 à 12, **caractérisé en ce qu'**un dispositif (17) de réduction de données est prévu de manière à réduire des données de la pression détectée (P) du manchon, auquel cas le dispositif de réduction de données comporte de préférence un dispositif (14) de détermination du point de base servant à déterminer un point de base (Fₙ) associé à un maximum respectif d'oscillation (Mₙ), et qu'un dispositif de mémoire (8) pour mémoriser le maximum respectif d'oscillation, le point de base associé et l'instant (tₙ), auquel le maximum d'oscillation a été détecté, est prévu en tant qu'ensemble de données.

14. Appareil de mesure de la pression sanguine selon la revendication 13, **caractérisé en ce que** le dispositif (14) de détermination du point de base comporte un dispositif (21) de détermination de valeurs moyennes servant à déterminer des valeurs moyennes (Pₘ) de valeurs de la pression du manchon, détectées avant et après une oscillation (Oₙ) et une unité de calcul (16) servant à calculer le point de base respectif (Fₙ) en fonction des valeurs moyennes.

15. Appareil de mesure de la pression sanguine selon au moins l'une des revendications 9 à 14, **caractérisé en ce que** le dispositif (18) de calcul du maximum comporte un dispositif de sélection (20) pour sélectionner des oscillations pour déterminer les droites de régression (R_{SYS}, R_{DIA}), auquel cas de préférence le dispositif de sélection comporte un dispositif de calcul d'une valeur d'espérance servant à calculer des valeurs d'espérance systolique et diastolique (A*) et un dispositif de calcul de zone servant à calculer une zone respectif (X_{SYS}, X_{DIA}) autour des valeurs d'espérance systolique et diastolique, notamment en fonction de la fréquence cardiaque.

16. Appareil de mesure de la pression sanguine selon au moins l'une des revendications 9 à 15, **caractérisé en ce que** le dispositif (23) de calcul de valeurs de la pression sanguine comporte un dispositif, qui détermine l'apparition d'une oscillation, qui caractérise une valeur de la pression sanguine systolique ou diastolique, à partir des deux droites de régression (R_{SYS}, R_{DIA}).
